# EUROPEAN PATENT APPLICATION

(11) **EP 2 607 371 A1**
(43) Date of publication of application: **26.06.2013**
(21) Application number: 11382399.1
(22) Date of filing: 23.12.2011
(51) Int. Cl.: C07J 41/00, C07J 43/00, C07J 75/00

(54) **Processes for the preparation of abiraterone and related compouds**

(71) Applicant: Crystal Pharma, S.A.U., 47151 Boecillo - Valladolid (ES)
(72) Inventor: Pérez Encabo, Alfonso, E-47011 Valladolid (ES); Turiel Hernández, José Ángel, E-47011 Valladolid (ES); Gallo Nieto, Francisco Javier, E-47151 Boecillo - Valladolid (ES); Lorente Bonde-Larsen, Antonio, E-47151 Boecillo - Valladolid (ES); Sandoval Rodríguez, Celso Miguel, E-47151 Boecillo - Valladolid (ES)
(74) Representative: ABG Patentes, S.L.

(57) **Abstract**

The present invention relates to processes for obtaining abiraterone and derivatives thereof, such as abiraterone acetate, by means of a C-C coupling through a steroid hydrazone of general formula (II) or a Suzuki coupling through a steroid borate of general formula (IV), as well as to intermediates useful in said processes.

## Description

### FIELD OF THE INVENTION

The present invention relates to processes for obtaining abiraterone and derivatives thereof, such as abiraterone acetate, as well as to intermediates useful in said processes.

### BACKGROUND

Abiraterone acetate [17-(3-pyridyl)-5,16-androstadien-3β-acetate] is a steroid compound which inhibits selectively and efficiently the enzyme 17-a-hydroxylase-C17-20-lyase, which catalyzes the conversion of dehydroepiandrosterone and androstenedione to testosterone. The inhibition of said enzyme causes a strong decrease of testosterone levels in the patient and therefore this drug is used in the treatment of certain hormone-dependent tumors resistant to chemotherapy such as prostate cancer. This compound has the following chemical formula:

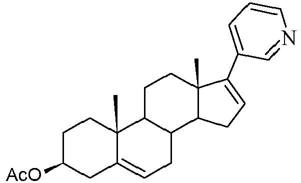

This product was disclosed for the first time in WO 93/20097, which also provides a synthetic process for its preparation including as last step the reaction of an enol triflate with a pyridine borate by Suzuki coupling (see scheme below). However, this process is not viable in practice, mainly because of the difficulty in preparing the enol trifluorosulfonate at the 17-position 2: this step, apart from proceeding with a poor conversion and low yield, gives place to the impurity tri-unsaturated 3 in a 10% yield, which only may be removed by column chromatography. Further, the product obtained after the subsequent Suzuki coupling must be also purified by column chromatography according to the examples provided therein.

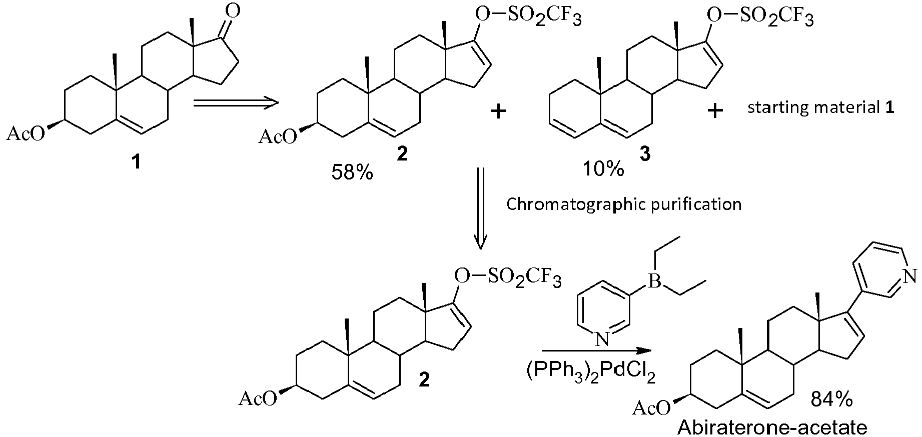

The above-mentioned impurity was prevented in later processes (EP 1 781 683 y EP 1 789 432) thanks to the use of alternative bases to that previously employed (i.e. 2,5-ditert-butyl-4-methylpyridine) such as DABCO, DBU or tryethylamine. However, in the sole example described in said documents, whilst the final product is achieved without using any column chromatography, it is obtained in a global yield of scarcely 21% and shows a purity of only 96.4%.

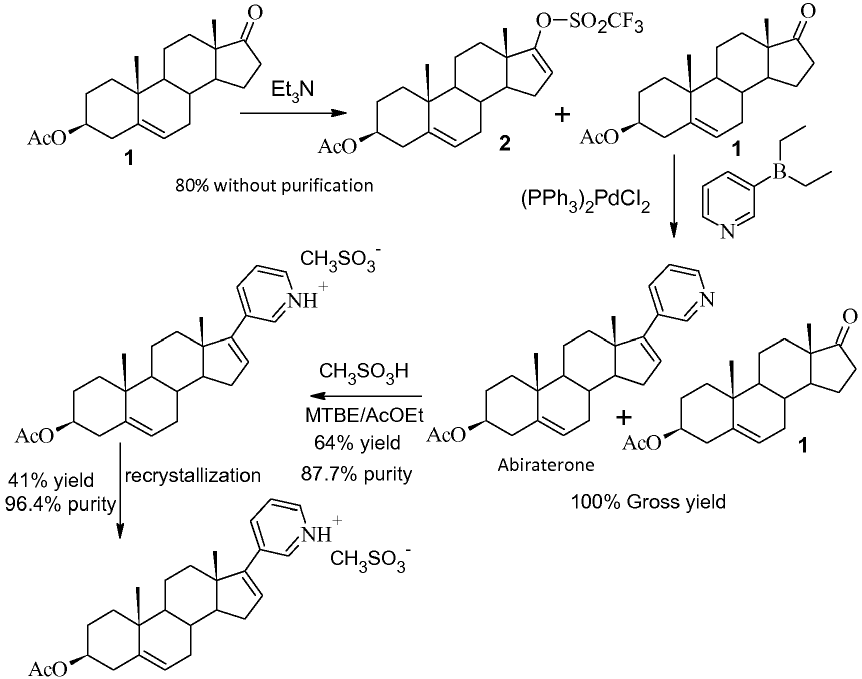

EP 0 721 461 proposes the use of a vinyl iodide or bromide intermediate instead of the enol triflate, as depicted in the following scheme:

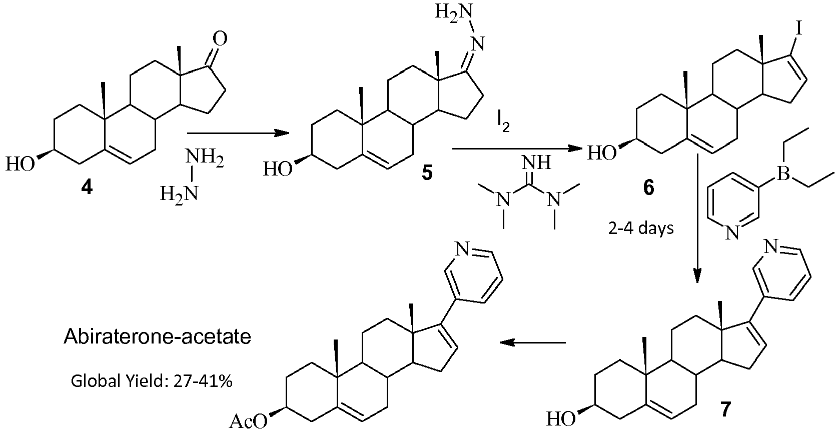

However, the iodo-enol is much less reactive than the triflate in the coupling with the pyridine borane, resulting in long reaction times (48 hours - 4 days) with a part of the starting material unreacted and wherein until a 5% of a dimeric impurity is obtained, which can only be removed by purification by means of reverse phase column chromatography:

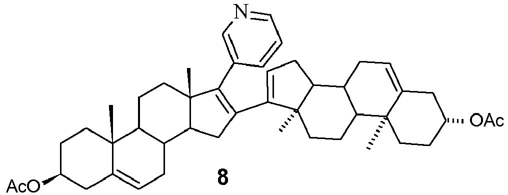

Therefore, there is still a need of developing new processes for obtaining 17-(3-pyridyl)-5,16-androstadien-3β-ol and related compounds, some of which are of therapeutic interest (e.g. abiraterone acetate) which overcome all or part of the drawbacks associated to the known processes belonging to the state of the art.

### BRIEF DESCRIPTION OF THE INVENTION

The present invention is faced with the problem of providing an alternative process for obtaining 17-(3-pyridyl)-5,16-androstadien-3β-ol and derivatives thereof, particularly abiraterone acetate, which solves the aforementioned drawbacks existing in the different synthesis of the state of the art.

The present invention provides various novel synthetic processes for obtaining abiraterone and its corresponding derivatives [encompassed under general formula (I)], enabling its preparation at an industrial scale in an advantageous way with respect to the processes disclosed so far.

In one aspect, the present invention refers to a process for the preparation of a compound of general formula (I) or a salt or solvate thereof, which comprises reacting a compound of general formula (II) with a compound of general formula (III) in presence of a palladium catalyst and a base. 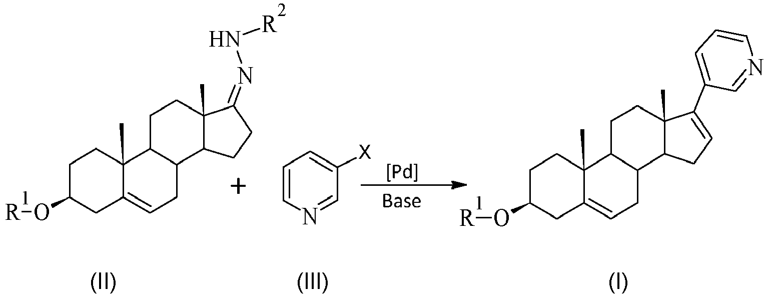 wherein the variables take the meanings indicated hereinafter.

In another aspect, the present invention refers to a process for the preparation of a compound of general formula (I) or a salt or solvate thereof, which comprises reacting a compound of general formula (IV) with a compound of general formula (III) in presence of a palladium catalyst and a base. 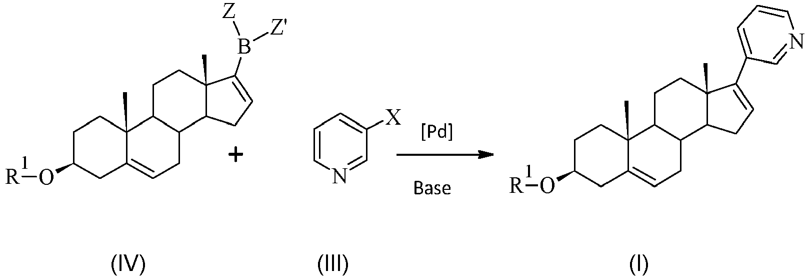 wherein the variables take the meanings indicated hereinafter.

In a further aspect, the present invention refers to the transformation and/or purification of a compound of general formula (I) obtained by any of the above processes in other compound of general formula (I) (specially abiraterone acetate) by any known process in the state of the art, preferably, through all or some of the following steps:
i) purification of a compound of formula (I) by means of crystallization and/or salt formation;
ii) transformation of the compound of formula (I) to abiraterone (R¹=H), by means of a deprotection reaction which, depending on the nature of group R¹, comprises:
   a) hydrolysis in acid or basic media,
   b) use of fluoride reagents, or
   c) oxidation or reduction;
iii) esterification of abiraterone (R¹=H) to afford abiraterone acetate.

In a further aspect, the present invention refers to compounds of general formula (II) or a salt or solvate thereof. In a further aspect, the present invention refers to compounds of general formula (IV) or a salt or solvate thereof. In a further aspect, the present invention refers to compounds of formula (I), wherein R¹ is SiR³R⁴R⁵, or a salt or solvate thereof.

In a further aspect, the present invention refers to a process for the preparation of a salt of a compound of formula (I), wherein R¹ is SiR³R⁴R⁵, by recovering the salt from a solution of the free base in any suitable solvent by treating the solution with an appropriate acid. Preferably the acid is hydrochloric acid.

These aspects and preferred embodiments thereof are additionally also defined hereinafter in the detailed description, as well as in the claims.

### DETAILED DESCRIPTION OF THE INVENTION

### Definitions

In the context of the present invention, the following terms have the meaning detailed below.

As used herein, the term "C₁-C₈ alkyl" relates to a radical derived from a linear or branched alkane, with 1 to 8 carbon atoms, for example, methyl, ethyl, propyl, butyl, etc., optionally substituted with one or more substituents independently selected from halogen, C₆-C₁₄ aryl and C₁-C₈ alkyl. An example of substituted alkyl is benzyl, which may be, in turn, substituted with methoxy, nitro, ciano, halo, phenyl, etc.

As used herein, the term "C₃-C₆ cycloalkyl" relates to a radical derived from a cycloalkane, with 3 to 6 carbon atoms, for example, cyclopropyl, cyclobutyl, etc., optionally substituted with one or more substituents independently selected from halogen, C₆-C₁₄ aryl and C₁-C₈ alkyl.

As used herein, the term "C₆-C₁₄ aryl" relates to a radical derived from an aromatic hydrocarbon, with 6 to 14 carbon atoms, for example, phenyl, tolyl, xylyl, naphthyl, etc., optionally substituted with one or more substituents independently selected from halogen and C₁-C₈ alkyl.

As used herein, the term "C₁-C₈ alkoxy" relates to an O-alkyl radical, with 1 to 8 carbon atoms, for example, methoxy, ethoxy, propoxy, butoxy, etc., optionally substituted with one or more substituents independently selected from halogen and C₁-C₈ alkyl.

As used herein, the term "halogen" or "halo" relates to fluorine, chlorine, bromine or iodine.

As used herein, the term "hydroxyl protecting group" (HPG) includes any group capable of protecting a hydroxyl group. Illustrative examples of hydroxyl protecting groups have been described by Green TW et al. in "Protective Groups in Organic Synthesis", 3rd Edition (1999), Ed. John Wiley & Sons (ISBN 0-471-16019-9). Virtually any hydroxyl protecting group can be used to put the invention into practice; nevertheless, in a particular embodiment, the hydroxyl protecting group is an ester group or an ether group, which can be converted into a hydroxyl group under mild conditions. Illustrative, non-limiting examples of HPGs include esters (COR), carbonates (COOR), amides (CONRR'), silyl radicals [Si(R³)(R⁴)(R⁵)], and ethers (R⁶), wherein
R and R' are independently selected from the group consisting of optionally substituted C₁-C₈ alkyl, optionally substituted C₃-C₆ cycloalkyl and optionally substituted C₆-C₁₄ aryl;
R³, R⁴ and R⁵ are independently selected from the group consisting of optionally substituted C₁-C₈ alkyl, optionally substituted C₃-C₆ cycloalkyl, optionally substituted C₆-C₁₄ aryl, optionally substituted C₁-C₈ alkoxy, and halogen; and
R⁶ is selected from the group consisting of optionally substituted C₁-C₈ alkyl, optionally substituted C₃-C₆ cycloalkyl and optionally substituted C₆-C₁₄ aryl.

Representative examples of esters, carbonates and amides as HPGs are those wherein R is methyl or benzyl.

Representative examples of amides as HPGs are those wherein R and/or R' is independently selected from methyl and benzyl.

Representative examples of silyl groups as HPGs are trimethylsilyl (TMS), dimethylphenylsilyl (DMPS) or dimethyl-t-butylsilyl (TBDMS).

Representative examples of ethers as HPGs are methyl ethers (CH₂OR⁸), ethyl ethers (CH₂CH₂OR⁸) and benzyl ethers, wherein R⁸ is C₁-C₈ alkyl, such as methoxymethyl, 1-ethoxyethyl.

The compounds used in the process described by the present invention can be obtained in free form or in solvate form. In both cases, they are preferably obtained in crystalline form, both as free compounds or as solvates (for example, hydrates, alcoholates, etc.), both forms being included within the scope of the present invention. Solvation methods are generally well known in the state of the art.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known by the persons skilled in the art. Such salts are generally prepared, for example, by reacting the free acid or base forms of said compounds with a stoichiometric amount of the suitable base or acid in water or in an organic solvent or in a mixture of the two. Nonaqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, *p*-toluenesulfonate, camphorsulfonate, etc. Illustrative examples of base addition salts include inorganic base salts such as, for example, ammonium salts and organic base salts such as, for example, ethylenediamine, ethanolamine, N,N-dialkylenethanolamine, triethanolamine, glutamine, amino acid basic salts, etc. Illustrative examples of metal salts include, for example, sodium, potassium, calcium, magnesium, aluminum and lithium salts.

The term "pharmaceutically acceptable" relates to molecular entities and compositions being physiologically tolerable and normally not causing an allergic reaction or similar adverse reaction, such as gastric discomfort, dizziness and the like, when they are administered to a human being. Preferably, as used in this description, the term "pharmaceutically acceptable" means approved by a governmental regulatory agency or listed in the US pharmacopoeia or another generally recognized pharmacopoeia for use in animals, and more particularly in humans.

For those persons skilled in the art, it will be evident that the scope of the present invention also includes salts which are not pharmaceutically acceptable as possible means for obtaining pharmaceutically acceptable salts.

Unless otherwise indicated, the compounds of the invention also include compounds which differ in the presence of one or more isotopically enriched atoms. By way of illustration, compounds having the structures defined herein, with the exception of the substitution of at least one hydrogen with a deuterium or with tritium, or the substitution of at least one carbon with a carbon enriched in ¹³C or ¹⁴C, or at least one nitrogen with a nitrogen enriched in ¹⁵N, are within the scope of this invention.

The term "complex" means a molecular structure in which neutral molecules or anions (called ligands) bond to a central metal atom (or ion) by coordinate covalent bonds. Extensive descriptions of terms related to coordination chemistry in reference books such as Robert H. Crabtree "The Organometallic Chemistry of the Transition Metals", Wiley-Interscience; 4 ed., 2005.

The term "catalyst" is recognized in the art and means a substance that increases the rate of a reaction without modifying the overall standard Gibbs energy change in the reaction and without itself being consumed in the reaction. The changing of the reaction rate by use of a catalyst is called catalysis. As used herein, the catalyst is used in a substoichiometric amount relative to a reactant, i. e. a catalytic amount. A general preferred catalytic amount is considered herein from 0.001 to 20 mol% of catalyst relative to the steroid compound to undergo coupling.

The term "ligand" refers to a molecule or ion that is bonded directly (i.e. covalently) to a metal center.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

### C-C coupling through a hydrazone of general formula (II)

Palladium-catalyzed C-C coupling reactions usually require the presence of a component with nucleophilic character represented by an organometallic compound such as organoboron (Suzuki), organozinc (Negishi), organostannane (Stille), etc. Recently, it has been found that certain hydrazones, in presence of a base, can act as nucleophiles in the same way without requiring any organometallic specie (A. Takemiya, J.F. Hartwig, J. Am.Chem.Soc., 2006, 128, 14800).

More recently, this methodology has been applied to the preparation of di- and trisubstituted olefins from tosyl-hydrazones in presence of a base, an aryl halide and a Pd catalyst (J. Barluenga et al, Angew. Chem. Int. Ed., 2007, 46, 5587-90 and J. Barluenga et al, Chem. Eur. J. 2008, 14, 4792-5).

In this context, the inventors have surprisingly and unexpectedly found that it is possible to extend this methodology to the preparation of vinyl-aryl(heteroaryl) derivatives such as the compounds of general formula (I), among which abiraterone is encompassed, by reaction of a steroid hydrazone of general formula (II) with a 3-substituted pyridine of general formula (III) in presence of a Pd(0) or Pd(II) catalyst and a base 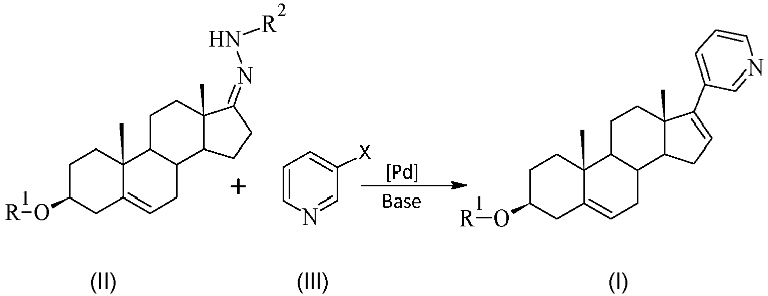 wherein
R¹ is selected from the group consisting of H and a hydroxyl protecting group (HPG);
R² is SO₂R⁷;
R⁷ is selected from the group consisting of optionally substituted C₁-C₈ alkyl and optionally substituted C₆-C₁₄ aryl; and
X is halogen or OSO₂CF₃.

As compared with the prior art approaches, neither the enolization of the ketone group at the 17-position nor the use of boron organometallic reagents are necessary in this process. Further, the impurities disclosed in the background of the invention for the prior procedures are not obtained, thus avoiding their purification. All this results in a cleaner and straighter process with better yields than those described so far for obtaining this kind of substrates. Moreover, as will be described hereinafter, it is possible to arrive at abiraterone and related compounds of general formula (I) from the starting ketone steroids without isolation of intermediate compounds, i.e., in a one-pot fashion.

The reaction of the compounds of general formula (II) with the compounds of general formula (III) is carried out under Pd catalysis. Examples of Pd catalysts that may be used include, without limitation, [Pd₂(dba)₃] (dba= trans, trans-dibenzylidene acetone), Pd(PPh₃)₄, PdCl₂(CNMe)₂, Pd(OH)₂ and Pd(OAc)₂ (Organic Letters 2010, 12 (18), 4042-4045). Preferably the catalyst is selected from Pd₂(dba)₃, Pd(PPh₃)₄ and PdCl₂(CNMe)₂, more preferably the Pd catalyst is Pd₂(dba)₃. Typically, the amount of the catalyst ranges from about 0.5% mol to about 10% mol, preferably from about 1% mol to about 6% mol.

Together to the Pd catalyst, ligands able to associate with the Pd atom have been used to facilitate the reaction so that a palladium complex is formed in the reaction media. In a preferred embodiment, the ligand is a phosphine ligand. Phosphine ligands are widely known by the skilled person since they are commonly used in organic catalysis. Illustrative, non-limiting examples of suitable ligands include X-phos (2-dicyclohexylphosphino-2',4',6'-triisopropylbiphenyl), dppp (1,4-bis(diphenylphosphino)butane), S-phos (2-dicyclohexylphosphino-2',6'-dimethoxybiphenyl), dppm (1,1-bis(diphenylphosphino)-methane), dippe (1,2-bis(diisopropylphosphino)ethane, dmpe (1,2-Bis(dimethylphosphino)ethane, dppe (1,2-bis(diphenylphophino)ethane, etc. Preferably the ligand is X-phos or dppp, more preferably X-phos. The amount of ligand used depends on the amount of palladium catalyst. In general, the amount of the ligand ranges from about 1% mol to about 20% mol, preferably from about 2% mol to about 12% mol.

The coupling is performed in basic medium. Alkoxides and carbonates of alkaline and alkaline earth metals have been found particularly useful such as for example alkoxides and carbonates of Li, Na, K and Cs. Illustrative, non-limiting examples of suitable bases for the coupling step include t-BuOLi, MeOLi, MeONa and C_{S}CO₃- In a preferred embodiment, the base is t-BuOLi. The amount of base preferably ranges from about 2 to about 20 eq for each equivalent of compound of formula (II), more preferably from about 4 to about 15 eq. In general, about 7.5 eq of base are suitable for the reaction occurs.

According to a particular embodiment, this coupling reaction is carried out in an organic solvent or mixture of solvents, for example, an ether (e.g., tetrahydrofuran (THF), 2-methyltetrahydrofuran, 1,2-dimethoxyethane (DME), dioxane, 1,3-dioxolane, etc.) or an aromatic solvent (e.g., toluene, xylene, etc.). In a more particular embodiment, the reaction of the compounds of formulas (II) and (III) is carried out in dioxane.

Likewise, the coupling reaction is suitably carried out under heating, for example at temperatures comprised between about 40°C and about 140°C, preferably between about 65°C and about 110°C, more preferably between about 80°C and about 110°C.

The compound of formula (III) is typically used in an amount ranging from about 1.1 and about 3 eq for each equivalent of compound of formula (II), preferably from about 1.3 to about 1.6 eq. A particular and preferred example of compound of formula (III) is 3-bromopyridine.

Compounds of formula (II) or a salt or solvate thereof are claimed *per se* in the present invention.

According to a particular embodiment, in the compound of formula (II) R¹ is selected from the group consisting of H, COMe, SitBuMe₂ (TBDMS) and SiMe₂Ph (DMPS). More preferably, R¹ is TBDMS.

According to another particular embodiment, in the compound of formula (II) R² is SO₂R⁷ wherein R⁷ is an optionally substituted C₆-C₁₄ aryl, such as Ph, Tol, 2,4,6-trimethylphenyl or 2,4,6-triisopropylphenyl.

The products of formula (I) obtained can be purified by column chromatography or preferably by means of industrially acceptable processes such as, for example, by means of a crystallization process, either of the product as a free base or, more preferably, through the formation of an addition salt. During the process of precipitation as a salt, the product is purified from all those impurities of neutral character. An illustrative, non-limiting example of suitable solvent for said crystallization is THF. Likewise, addition salts of HCl are preferred.

In a especially preferred embodiment, 3-(tert-butyldimethylsiloxy)-17-p-toluenesulfonylhydrazone-androsta-5-en is subject to reaction with 6% mol Pd₂(dba)₃, 12% mol X-Phos, 7.5 eq tBuOLi and 1.5 eq 3-bromopyridine in 20-30 volume of dioxane at a temperature between 90 and 100 °C for 6-15 hours. Then, the reaction mixture is filtered and the solvent evaporated. The residue obtained is purified by redissolution in THF and precipitation of the product obtained as an acid addition salt of HCl (using HCl aq 1-2 M) at room temperature. Thus, the salt obtained can be used directly or can be neutralized to obtain the product in neutral form. This process for obtaining chlorhydrate of 3-(tert-butyldimethylsiloxy)-17-(3-pyridyl)-androsta-5-en is depicted below:

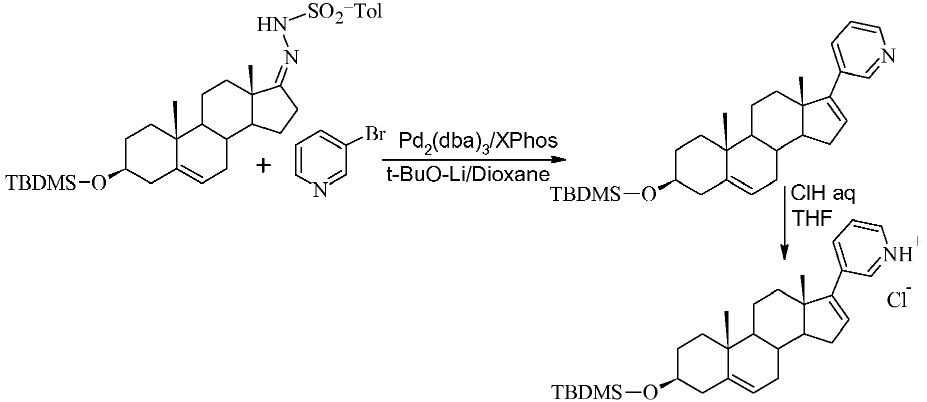

The compounds of general formula (II) can be readily prepared from a ketone of general formula (VI) and a hydrazine of general formula (VII):

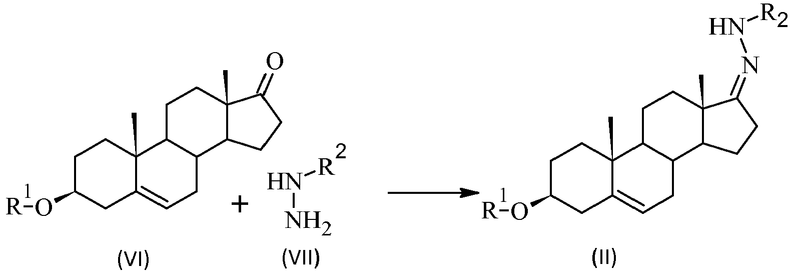

This reaction can be conveniently carried out by mixing both components in equimolecular amounts or with an excess of hydrazine (e.g. about 10%), preferably at 40°C-110°C, in a suitable solvent. From an experimental point of view, it is preferred to use the same solvent than that employed in the subsequent step such as dioxane, THF, etc., (J. Barluenga et al, Chem. Eur. J. 2008, 14, 4792-5). Further, the reaction can be catalyzed by using about 0.05-0.1 eq TsOH or any other suitable acid which speeds the reaction. Under catalysis, the process typically undergoes in about two hours at 50-90 °C.

The hydrazones thus obtained, optionally, can be isolated by precipitation by addition over water or by solvent evaporation and precipitation of the residue by addition of diethyl ether, toluene, heptane or any other suitable solvent, depending on the nature of R¹.

It has been found that the condensation of a ketone of general formula (VI) and a hydrazine of general formula (VII), depending on the nature of R¹, may proceed with the hydrolysis of the hydroxyl protecting group on position 3 to some extent. Accordingly, in some instances, said condensation is initially carried out with 5-dehydroepiandrosterone (5-DHEA) to obtain the corresponding hydrazone (II-H), which may be subsequently protected on its hydroxyl group through common processes known in the state of the art (see scheme below).

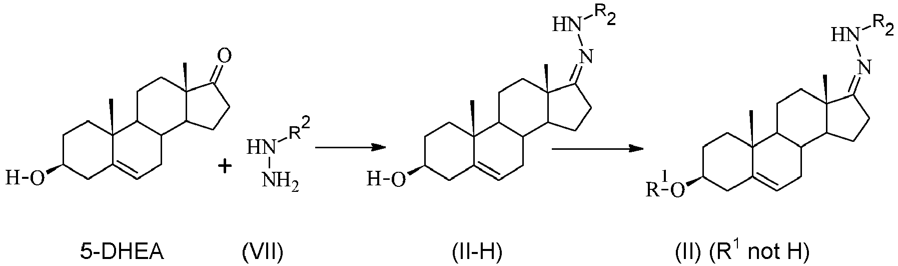

According to a preferred embodiment, the formation of the hydrazone of general formula (II) may be carried out in THF with 0.1 eq of TsOH and 1.1 eq of p-toluenesulfonyl hydrazide at 50°C-90°C. After completion, the reaction mixture is poured into water under stirring to afford a solid, which is subsequently filtered.

The easiness of this process opens the possibility that the compounds of general formula (I) can be also obtained directly from the compounds of general formula (VI), without isolation of the intermediate compounds (II), i.e. in a one-pot fashion, as depicted below.

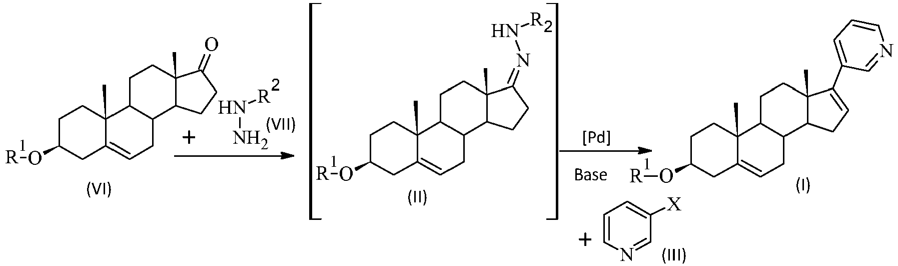

### Suzuki coupling through a organoboron steroid compound (IV)

After an extensive research on processes for the preparation of abiraterone and related compounds, the inventors have surprisingly found that is easier and more convenient to carry out a Suzuki type coupling, in which the organoboron compound is situated on the steroid and the electrophilic compound is the 3-substituted pyridine, conversely to the processes disclosed previously in the state of the art. In a further aspect, the present invention is directed to the preparation of a compound of general formula (I) or a salt or solvate thereof, by reacting a steroid borate of general formula (IV) with a 3-substituted pyridine of general formula (III) in presence of a Pd(0) or Pd(II) catalyst and a base. 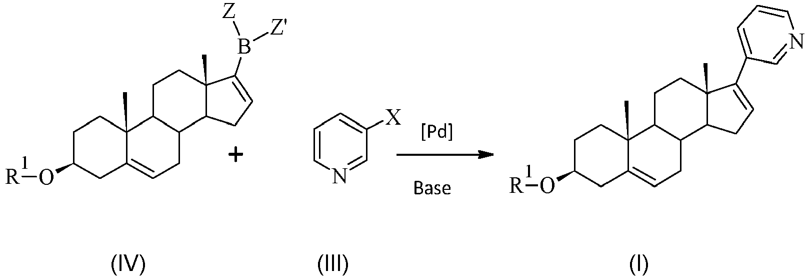 wherein
R¹ is selected from the group consisting of H and a hydroxyl protecting group (HPG);
X is halogen or OSO₂CF₃; and
Z and Z' are independently selected from the group consisting of hydroxyl, optionally substituted C₁-C₈ alkoxy and optionally substituted C₁-C₈ alkyl, or
Z and Z' together form an optionally substituted C₂-C₃ alkylenedioxy group.

The reaction of the compounds of general formula (IV) with the compounds of general formula (III) is carried out under Pd catalysis, for example in presence of a Pd(0) catalyst having phosphine ligands such as Pd(PPh₃)₄ or a PD(II) catalyst having phosphine ligands, which is reduced *in situ* to Pd(0) such as Pd(PPh₃)₂Cl₂. Typically, the amount of the catalyst is about 0.2% mol to about 6% mol, preferably about 1-2% mol.

The process requires the presence of a base. Preferably, the base is selected from sodium, cesium and potassium carbonate. Further, the reaction preferably proceeds in presence of water either in a homogenous system or a biphasic system, the latter being preferred.

According to a particular embodiment, this coupling reaction is carried out in an organic solvent or mixture of solvents, for example, an ether (e.g., tetrahydrofuran (THF), 2-methyltetrahydrofuran, 1,2-dimethoxyethane (DME), dioxane, 1,3-dioxolane, etc.) or an aromatic solvent (e.g., toluene, xylene, etc.). In a more particular embodiment, the reaction of the compounds of formulas (IV) and (III) is carried out in an organic solvent, preferably THF, in the presence of variable amounts of water; the amount of water typically ranges from about 10% to about 100% with respect to the amount of the organic solvent/s employed.

Likewise, the coupling reaction is suitably carried out under heating, for example at temperatures comprised between about 40°C and about 110°C, preferably between about 60°C and about 90°C.

The compound of formula (III) is typically used in an amount ranging from about 1.1 and about 3 eq for each equivalent of compound of formula (II), preferably from about 1.3 to about 1.6 eq. A particular and preferred example of compound of formula (III) is 3-bromopyridine.

Compounds of formula (IV) or a salt or solvate thereof are claimed *per se* in the present invention.

According to a particular embodiment, in the compound of formula (IV) R¹ is selected from the group consisting of H, COMe, SitBuMe₂ (TBDMS) and SiPhMe₂ (DMPS). More preferably, R¹ is TBDMS.

According to another particular embodiment, in the compound of formula (IV) Z and Z' corresponds with OMethyl groups or, together, form an ethylendioxygroup.

Once the reaction is complete, inorganic salts may be removed by extraction with water and an organic solvent such as dichloromethane, toluene, diethyl ether, cyclopentylmethyl ether, ethyl acetate or any other suitable solvent.

The product contained in the organic phase can, depending on the nature of R¹, be isolated by solvent removal and recrystallization of the residue in a suitable solvent, or alternatively, by precipitation in the form of a salt, such as chlorhydrate, sulfate, methanesulfonate, etc., or a combination of both methods.

Boron derivatives of general formula (IV) may be obtained from hydrazones of general formula (IIa) through the following sequence (*J.Am.Chem.Soc*., **2008**, 130, 8481-8490):

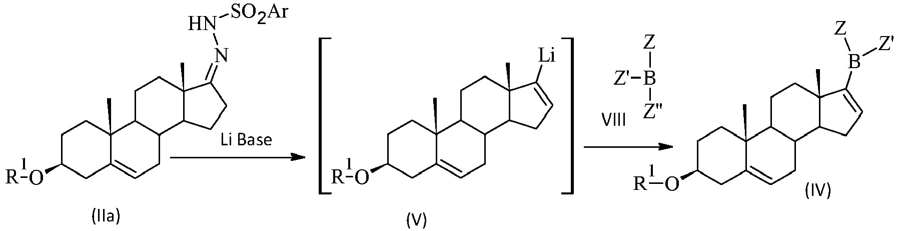

Compounds of general formula (IIa) may be prepared in turn from ketones of general formula (VI), similarly to the compounds of general formula (II).

Enol lithium compounds of general formula (V) may be prepared by Shapiro reaction, from hydrazones of formula (IIa) and a lithium base, including common alkyl lithium bases such as Bu-Li, Hexyl-Li, tert-Butil-Li, etc. Then, the enol lithium compound reacts with a borate of general formula (VIII) to afford a vinyl-borate of general formula (IV).

In a borate of general formula (VIII) Z, Z' and Z" are independently selected from the group consisting of hydroxyl, optionally substituted C₁-C₈ alkoxy and optionally substituted C₁-C₈ alkyl, or Z and Z' together form a optionally substituted C₂-C₃ alkylenedioxy group and Z" is selected from the group consisting of hydroxyl, optionally substituted C₁-C₈ alkoxy and optionally substituted C₁-C₈ alkyl.

Alternatively, the borate compound of general formula (IV) may be generated from the corresponding iodo or bromo vinyl derivative according to the following scheme (J. Org. Chem., 1985, 50, 2438-43): 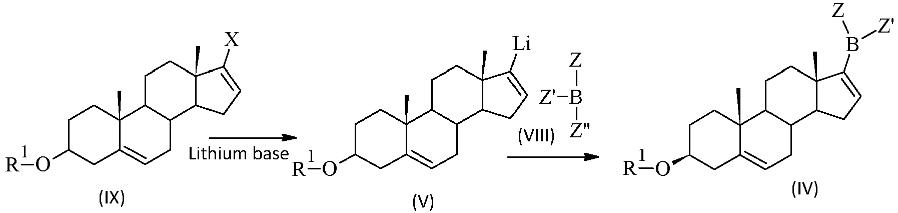 wherein
R¹ is selected from the group consisting of H and a hydroxyl protecting group (HPG);
X is bromo or iodo;
Z, Z' and Z" are independently selected from the group consisting of hydroxyl, optionally substituted C₁-C₈ alkoxy and optionally substituted C₁-C₈ alkyl, or
Z and Z' together form a optionally substituted C₂-C₃ alkylenedioxy group and Z" is selected from the group consisting of hydroxyl, optionally substituted C₁-C₈ alkoxy and optionally substituted C₁-C₈ alkyl.

A preferred lithium base in the above process is t-BuOLi.

### Transformation and/or purification of compounds of general formula (I)

In a further aspect, the invention refers to the transformation and/or purification of compounds of general formula (I) obtained by any of the above processes in other compounds of general formula (I) by any known process in the state of the art, through all or some of the following steps:
i) purification of a compound of formula (I) by means of crystallization and/or salt formation;
ii) transformation of the compound of formula (I) to abiraterone (R¹=H), by means of a deprotection reaction which, depending on the nature of group R₁, comprises:
   a) hydrolysis in acid or basic media,
   b) use of fluoride reagents, or
   c) oxidation or reduction;
iii) esterification of abiraterone (R¹=H) to afford abiraterone acetate.

### • Step i) purification of a compound of formula (I) by means of crystallization and/or salt formation

The products of formula (I) obtained can additionally be purified by column chromatography or preferably by means of industrially acceptable processes such as, for example, by means of a crystallization process, either of the product as a free base or, more preferably, through the formation of an addition salt. During the process of precipitation as a salt, the product is purified from all those impurities of neutral character. An illustrative, non-limiting example of suitable solvent for said crystallization is THF. Likewise, addition salts of HCl are preferred. The salt obtained in this way may be used directly or may be neutralized.

Particular compounds of formula (I) which may be isolated as a chlorhydrate salt by precipitation of the same by addition of an aqueous solution of chloride acid are those wherein R¹ is selected from H, COMe, TMS, DMPS and TBDMS.

### • Step ii) transformation of the compound of formula (I) to abiraterone (R¹=H)

Abiraterone can be prepared from a compound of general formula (I) wherein R¹ is a hydroxyl protecting group by conventional methods of deprotection known by persons skilled in the art (Green TW et al. in "Protective Groups in Organic Synthesis", 3rd Edition (1999), Ed. John Wiley & Sons (ISBN 0-471-16019-9).

For example, compounds of formula (I) wherein R¹ represents an ester (COR), a carbonate (COOR) or an amide (CONRR') can be easily converted into abiraterone by hydrolysis in basic or acid media according to well-established procedures of the state of the art.

Compounds of formula (I) wherein R¹ represents a silyl radical (SiR³R⁴R⁵) can be easily converted into abiraterone by the use of fluoride reagents such as fluoride salts or HF, acid media, oxidizing media, etc.

According to a particular embodiment, when R¹ = TBDMS, the deprotection conditions are: the silyl derivative is solved in THF and tetrabutylammonium fluoride in THF (1 M) is added at room temperature. The progress of this reaction can be easily monitored by TLC. The alcohol is isolated.

Compounds of formula (I) wherein R¹ represents an ether (R⁶) can be easily converted into abiraterone by hydrolysis in acid media (for example, for methyl ethers (CH₂OR⁸)), hydrogenation (for example, for benzyl ethers), oxidation (for example, for aryl ethers), etc.

### • Step iii) Esterification of abiraterone (R¹=H) to afford abiraterone acetate

The esterification of abiraterone to its acetate may be performed according to conventional chemical processes known by the persons skilled in the art. According to a particular embodiment, this esterification is carried out by using acetyl chloride as acylating agent and pyridine as solvent, or acetyl chloride as acylating agent and ethyl ether as solvent in presence of DMAP as catalyst (EP 0721461 B, US 5,618,807 A).

### Compounds of general formula (I) wherein R¹ is SiR³R⁴R⁵

In a further aspect, the present invention refers to compounds of formula (I), wherein R¹ is SiR³R⁴R⁵, or a salt or solvate thereof. 3-((Tert-butyldimethylsilyl)oxy)abiraterone (3-TBDMS-abiraterone) and its chlorhydrate salt represent preferred compounds of the invention.

Salts of silyl ethers of a compound of formula (I) may be recovered from a solution of the free base in any suitable solvent, or mixture of solvents, by treating the solution with the corresponding acid. Suitable solvents include esters and ethers. Esters which may be used include esters withy acetic acid, such as methyl acetate, ethyl acetate and isopropyl acetate. Ethers which may be used include diethyl ether, diisopropyl ether, methyl tert-butyl ether (MTBE), and especially tetrahydrofuran (THF), which gave a particularly good recovery of the salt. Preferably the acid is hydrochloric acid, such as HCl aq (1M).

According to a preferred embodiment, the chlorhydrate salt of 3-TBDMS-abiraterone is prepared by recovering the salt from a solution of the free base in any suitable solvent, more preferably THF, by treating the solution with hydrochloric acid.

In additional preferred embodiments, the preferences described above for the different substituents in the intermediates and compounds of the invention as well as for the conditions of the processes for their preparation are combined. The present invention is also directed to such combinations of preferred substitutions in the chemical formulae above and conditions of the processes for obtaining the same.

The following examples are merely illustrative of certain embodiments of the invention and cannot be considered as restricting it in any way.

### EXAMPLES

### Example 1.- Synthesis of 5-dehydroepiandrosterone p-toluenesulfonyl hydrazone

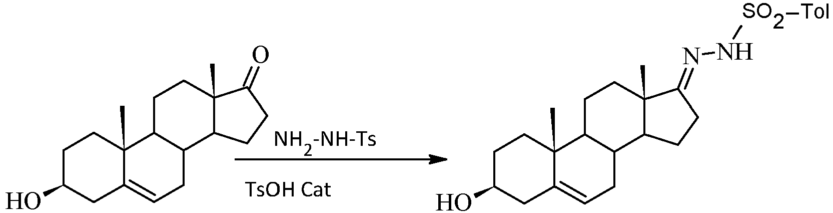

In a round bottom flask under inert atmosphere were added consecutively 10 g (34.67 mmol) of 5-DHEA, 7.32 g of NH₂NHTs (38.13 mmol), 0.66 g (10 %) of p-toluensulfonic acid and 70 ml of THF. The solution obtained was heated to boiling point for about 2-3 hours until the reaction was complete by TLC. The reaction mixture was cooled and the solvent evaporated under reduced pressure to afford a solid residue, which was resuspended in ethyl ether and filtered to yield 15.6 g of the corresponding 5-dehydroepiandrosterone p-toluenesulfonyl hydrazone (98% yield). Characteristic NMR signals:

¹H NMR (400 MHz, CDCl₃) δ 7.82 (d, J = 8.3 Hz, 2H), 7.29 (d, J = 8.1 Hz, 2H), 5.33 (d, J = 5.1 Hz, 1H), 3.51 (s, 1H), 2.42 (s, 3H), 1.00 (s, 3H), 0.82 (s, 3H).

### Example 2.- Synthesis of 5-dehydroepiandrosterone-2,4,6-trimethylbenzenesulfonyl hydrazone

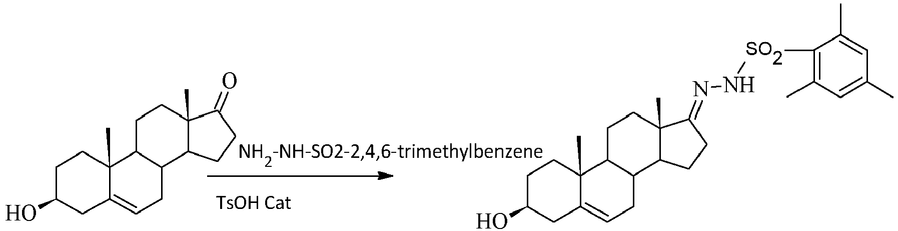

In a round bottom flask under inert atmosphere were added consecutively 2 g (6.93 mmol) of 5-DHEA, 1.63 g of 2,4,6-trimethylbenzenesulfonyl hydrazine (7.62 mmol), 0.133 g (10 %) of p-toluensulfonic acid and 30 ml of THF. The solution obtained was heated to boiling point for about 2-3 hours until the reaction was complete by TLC. The reaction mixture was cooled and the solvent evaporated under reduced pressure to afford a solid residue, which was resuspended in methanol and filtered to yield 3 g of the corresponding 5-dehydroepiandrosterone-2,4,6-trimethylbenzenesulfonyl hydrazone (89% yield). Characteristic NMR signals:

¹H NMR (400 MHz, CDCl₃) δ 7.02 (s, 1H), 6.94 (s, 2H), 5.33 (d, J = 5.2 Hz, 1H), 3.51 (s, 1H), 2.66 (s, 6H), 2.29 (s, 3H), 1.00 (s, 3H), 0.76 (s, 3H).

### Example 3.- Synthesis of 5-dehydroepiandrosterone benzenesulfonyl hydrazone

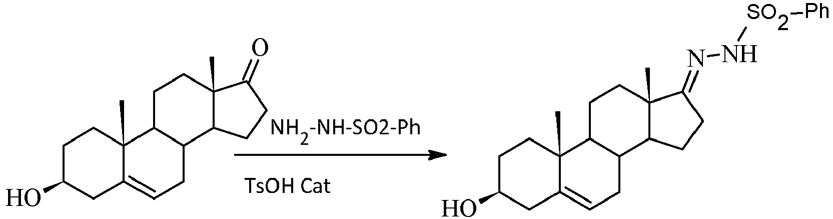

In a round bottom flask under inert atmosphere were added consecutively 2 g (6.93 mmol) of 5-DHEA, 1.43 g of NH₂NHSO₂-Ph (8.31 mmol), 0.133 g (10 %) of p-toluensulfonic acid and 30 ml of THF. The solution obtained was heated to boiling point for about 2-3 hours until the reaction was complete by TLC. The reaction mixture was cooled and the solvent evaporated under reduced pressure to afford a solid residue, which was resuspended in ether and filtered to yield 2.75 g of the corresponding 5-dehydroepiandrosterone benzenesulfonyl hydrazone (89% yield).

### Example 4.- Synthesis of 5-dehydroepiandrosterone 2,4,6-tri-isopropylbenzenesulfonyl hydrazone

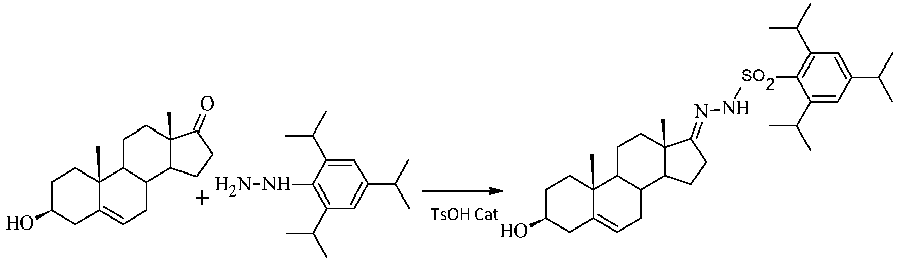

In a round bottom flask under inert atmosphere were added consecutively 0.4 g (1.39 mmol) of 5-DHEA, 0.45 g of 2,4,6-tri-isopropylbenzenesulfonyl hydrazine (1.52 mmol), 0.026 g (10 %) of p-toluensulfonic acid and 5 ml of THF. The solution obtained was heated to boiling point for about 2-3 hours until the reaction was complete by TLC. The reaction mixture was cooled and the solvent evaporated under reduced pressure to afford a solid residue, which was resuspended in heptane and filtered. The mother liquors were partially concentrated and cooled, affording a precipitate which was filtered again. The solids combined and dried yield 0.53 g of the corresponding dehydroepiandrosterone 2,4,6-tri-isopropylbenzenesulfonyl hydrazone (67% yield).

### Example 5.- Synthesis of 3-acetyl-5-dehydroepiandrosterone benzenesulfonyl hydrazone

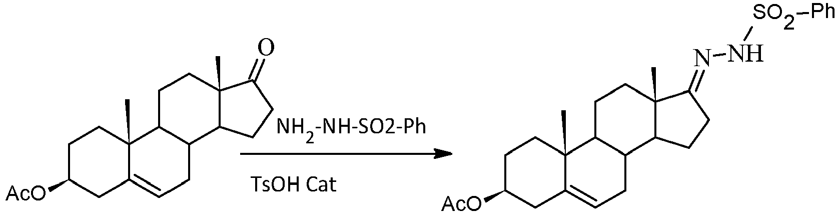

In a round bottom flask under inert atmosphere were added consecutively 15 g (45.39 mmol) of 5-DHEA-acetate, 9.38 g of NH₂NHSO₂-Ph (54 mmol), 0.86 g (10 %) of p-toluensulfonic acid and 60 ml of THF. The solution obtained was heated to boiling point for about 2-3 hours until the reaction was complete by TLC. The reaction mixture was cooled and the solvent was evaporated under reduced pressure to afford a solid residue, which was resuspended in ether and filtered to yield 20.80 g of the corresponding 3-acetyl-5-dehydroepiandrosterone benzenesulfonyl hydrazone (94% yield), along with a 10% of the alcohol compound as an impurity, resulting from a partial hydrolysis of the acetate group under the reaction conditions. Characteristic NMR signals:

¹H NMR (400 MHz, CDCl₃) δ 7.95 (dd, J = 8.4, 1.2 Hz, 2H), 7.58 (d, J = 7.5 Hz, 1H), 7.55 - 7.43 (m, 2H), 7.15 (s, 1H), 5.35 (d, J = 5.0 Hz, 1H), 4.66 - 4.51 (m, 1H), 3.47 (d, J = 31.6 Hz, 1H), 2.03 (s, 3H),1.02 (s, 3H), 0.79 (s, 3H).

### Example 6.- Syntesis of 3-((tert-butyldimethylsilyl)oxy)-5-dehydroepiandrosterone p-toluenesulfonyl hydrazone

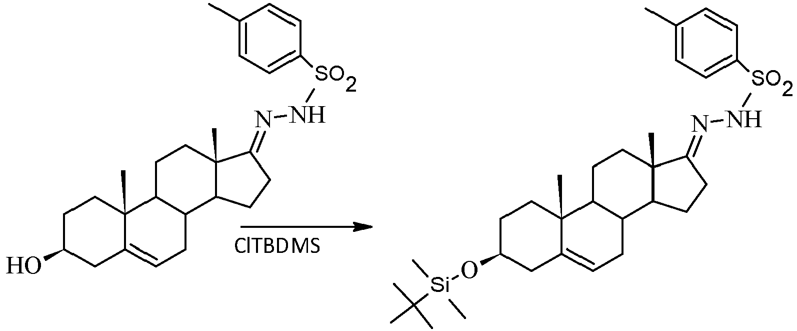

Over a suspension of 10 g (21.90 mmol) of 5-dehydroepiandrosterone p-toluenesulfonyl hydrazone and 50 ml of CH₂Cl₂ in a round bottom flask under inert atmosphere were added 7.15 g (4.8 eq) of imidazol. The reaction mixture was stirred at room temperature until a yellowish solution was obtained (about 10 min). Over the solution obtained 4.42 g (1.3 eq) of TBDMSiCl were added, maintaining stirring for 2 hours more until the reaction was complete by TLC.

The solvent was evaporated under reduced pressure to afford an oily residue. 20 ml of acetone and 100 ml of water were consecutively added and the mixture was stirred, affording a white solid as precipitate.

100 ml of water were added again over the suspension and it was filtered. The solid obtained was washed with water and dried under reduced pressure to yield 10.93 g of the corresponding 3-((tert-butyldimethylsilyl)oxy)-5-dehydroepiandrosterone p-toluenesulfonyl hydrazone (88% yield). Characteristic NMR signals:

¹H NMR (400 MHz, CDCl₃): δ 7.83 (d, J = 8.3 Hz, 2H), 7.29 (d, J = 8.1 Hz, 2H), 5.29 (d, J = 5.0 Hz, 1H), 3.46 (d, J = 4.7 Hz, 1H), 2.42 (s, 3H), 1.00 (s, 3H), 0.88 (s, 9H), 0.78 (s, 3H), 0.05 (s, 6H).

### Example 7.- Synthesis of 3-((tert-butyldimethylsilyl)oxy)-5-dehydroepiandrosterone 2,4,6-trimethylbenzenesulfonyl hydrazone

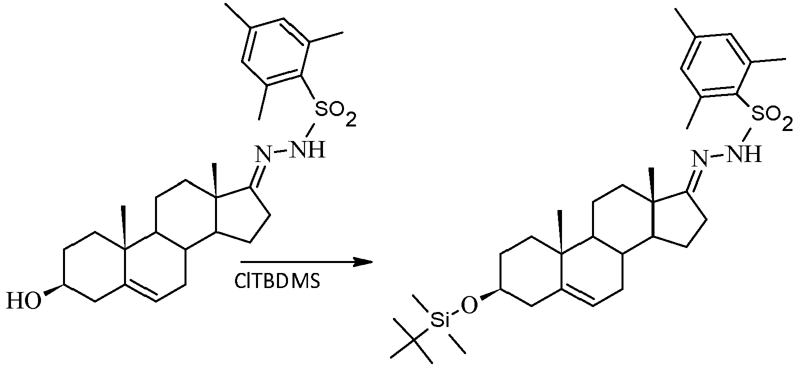

Over a suspension of 1 g (2.06 mmol) of 5-dehydroepiandrosterone-2,4,6-trimethylbenzenesulfonyl hydrazone and 20 ml of CH₂Cl₂ in a round bottom flask under inert atmosphere were added 0.67 g (4.8 eq) of imidazol. The reaction mixture was stirred at room temperature until a yellowish solution was obtained (about 10 min). Over the solution obtained 0.62 g (2 eq) of TBDMSiCl were added, maintaining stirring for 3 hours more until the reaction was complete by TLC.

The solvent was evaporated under reduced pressure to afford a solid residue, which was resuspended in water. Then, it was filtered, washed with water and dried under reduced pressure to yield 1.19 g of the corresponding 3-((tert-butyldimethylsilyl)oxy)-5-dehydroepiandrosterone 2,4,6-trimethylbenzenesulfonyl hydrazone (96% yield). Characteristic NMR signals:

¹H NMR (400 MHz, CDCl₃): δ 6.94 (d, J = 4.0 Hz, 3H), 5.30 (s, 1H), 3.53 - 3.40 (m, 1H), 2.67 (s, 6H), 2.30 (s, 3H), 0.99 (s, 3H), 0.88 (s, 9H), 0.75 (s, 3H), 0.05 (s, 6H).

### Example 8.- Synthesis of 3-((tert-butyldimethylsilyl)oxy)-5-dehydroepiandrosterone benzenesulfonyl hydrazone

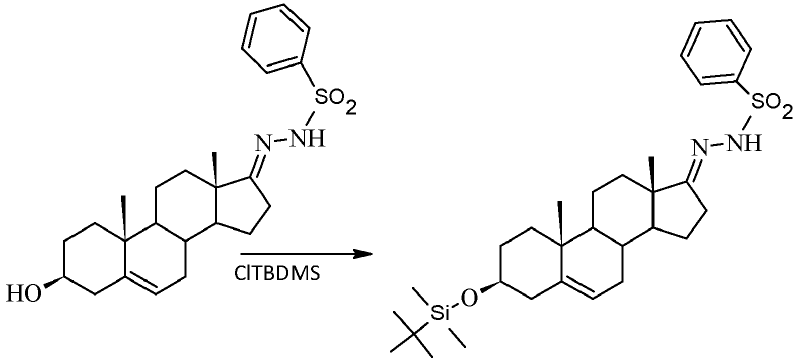

Over a suspension of 4 g (9.04 mmol) of 5-dehydroepiandrosterone benzenesulfonyl hydrazone and 50 ml of CH₂Cl₂ in a round bottom flask under inert atmosphere were added 2.95 g (4.8 eq) of imidazol. The reaction mixture was stirred at room temperature until a yellowish solution was obtained (about 10 min). Over the solution obtained 2.11 g (1.5 eq) of TBDMSiCl were added, maintaining stirring for 3 hours more until the reaction was complete by TLC.

The solvent was evaporated under reduced pressure to afford an oily residue. 8 ml of acetone and 50 ml of water were consecutively added and the mixture was stirred, affording a white solid as precipitate.

The suspension was filtered and the solid obtained was washed with water and dried under reduced pressure to yield 4.79 g of the corresponding 3-((tertbutyldimethylsilyl)oxy)-5-dehydroepiandrosterone benzenesulfonyl hydrazone (93% yield). Characteristic NMR signals:

¹H NMR (400 MHz, CDCl₃): δ (dd, J = 8.4, 1.3 Hz, 2H), 7.65 - 7.41 (m, 3H), 7.14 (s, 1H), 5.29 (d, J= 5.1 Hz, 1H), 3.46 (s, 1H), 1.00 (s, 3H), 0.90 - 0.87 (m, 9H), 0.78 (s, 3H), 0.05 (s, 7H).

### Example 9.- Synthesis of 3-((tert-butyldimethylsilyl)oxy)-5-dehydroepiandrosterone 2,4,6-tri-isopropylbenzenesulfonyl hydrazone

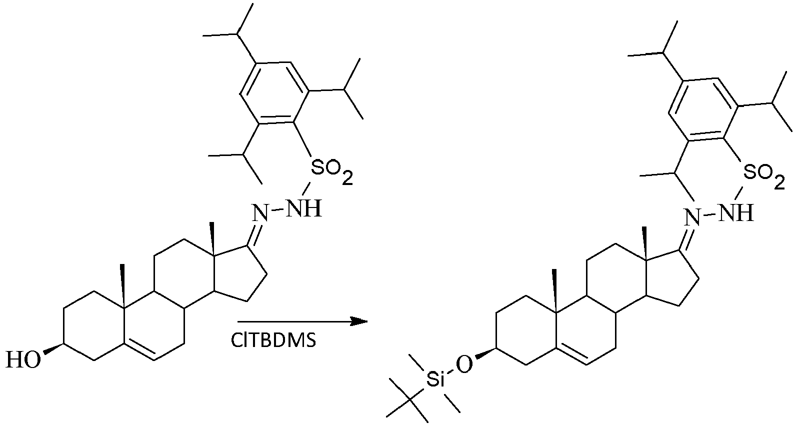

Over a suspension of 0.5 g (0.88 mmol) of 5-dehydroepiandrosterone-2,4,6-tri-isopropyllbenzenesulfonyl hydrazone and 15 ml of CH₂Cl₂ in a round bottom flask under inert atmosphere were added 0.29 g (4.8 eq) of imidazol. The reaction mixture was stirred at room temperature until a yellowish solution was obtained (about 10 min). Over the solution obtained 0.2 g (1.5 eq) of TBDMSiCl were added, maintaining stirring for 3 hours more until the reaction was complete by TLC.

The solvent was evaporated under reduced pressure to afford an oily residue. 1 ml of acetone and 25 ml of water were consecutively added and the mixture was stirred, affording a white solid as precipitate.

The suspension was filtered and the solid obtained was washed with water and dried under reduced pressure to yield 0.5 g of the corresponding 3-((tertbutyldimethylsilyl)oxy)-5-dehydroepiandrosterone 2,4,6-triisopropylbenzenesulfonyl hydrazone (73% yield). Characteristic NMR signals:

¹H NMR (400 MHz, CDCl₃): δ 7.15 (s, 2H), 6.94 (s, 1H), 5.48 - 5.42 (m, 1H), 5.30 (d, J = 5.1 Hz, 1H), 4.27-4.11 (m, 2H), 3.98-3.96 (m, 1H), 3.46 (s, 1H), 2.90 (s, 1H), 1.26 (t, J = 6.1 Hz, 18H), 0.98 (s, 3H), 0.88 (s, 9H), 0.74 (s, 3H), 0.05 (s, 6H).

### Example 10.- Synthesis of 3-((tert-butyldimethylsilyl)oxy)-5-dehydroepiandrosterone

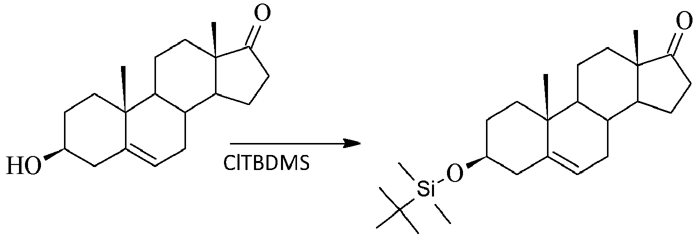

Over a suspension of 2 g (6.93 mmol) of 5-dehydroepiandrosterone and 20 ml of CH₂Cl₂ in a round bottom flask under inert atmosphere were added 2.26 g (4.8 eq) of imidazol. The reaction mixture was stirred at room temperature until a yellowish solution was obtained (about 10 min). Over the solution obtained 1.4 g (1.3 eq) of TBDMSiCl were added, maintaining stirring for 3 hours more until the reaction was complete by TLC.

The solvent was evaporated under reduced pressure to afford an oily residue. 5 ml of acetone and 40 ml of water were consecutively added and the mixture was stirred, affording a white solid as precipitate.

The suspension was filtered and the solid obtained was washed with water and dried under reduced pressure to yield 2.33 g of the corresponding 3-((tertbutyldimethylsilyl)oxy)-5-dehydroepiandrosterone (83% yield). Characteristic NMR signals:

¹H NMR (400 MHz, CDCl3): δ 5.34 (d, J = 5.1 Hz, 1H), 3.48 (d, J = 4.8 Hz, 1H), 1.02 (s, 3H), 0.88 (d, J = 2.9 Hz, 12H), 0.06 (s, 6H).

### Example 11.- Synthesis of abiraterone

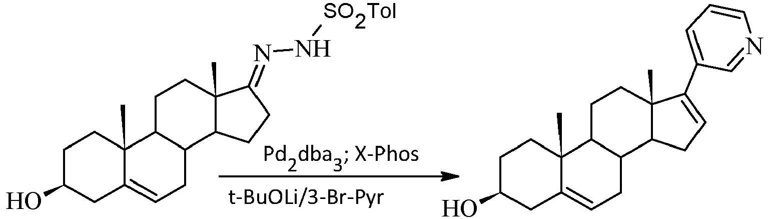

In a round bottom flask under inert atmosphere were added consecutively: 0.235 g (0.5 mmol) of 5-dehydroepiandrosterone p-toluenesulfonyl hydrazone, 0.0092 g (0.01 mmol, 2 %) of Pd₂(dba)₃, 0.0095 g (0.02 mmol, 4 %) of XPhos and 0.264 g (1.65 mmol, 3,3 eq) of ^{t}BuOLi. Thereafter 6 ml of dry dioxane and 0.067 ml (0.7 mmol, 1.4 eq) of 3-bromopyridine were also added. The suspension obtained was heated at reflux for 4 hours, and then it was cooled and filtered, washing the insoluble residue with THF (3 x 5 ml). The filtrates were combined and the solvent was evaporated under reduced pressure. In the residue obtained, abiraterone was detected as confirmed by NMR.

¹H NMR (400 MHz, CDCl₃): δ 8.62 (d, J = 1.7 Hz, 1H), 8.46 (dd, J = 4.8, 1.4 Hz, 1H), 7.64 (dd, J = 5.9, 4.0 Hz, 1H), 7.24 - 7.19 (m, 1H), 6.00 (dd, J = 3.2, 1.8 Hz, 1H), 5.46 - 5.31 (m, 1H), 3.54 (ddd, J = 15.5, 11.0, 4.3 Hz, 1H), 1.07 (s, 3H), 1.05 (s, 3H), -0.00 (s, 2H).

### Example 12.- Synthesis of abiraterone acetate from 3-acetyl-5-dehydroepiandrosterone toluenesulfonyl hydrazone

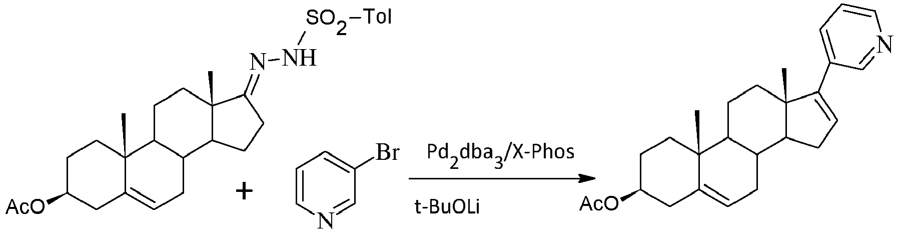

In a round bottom flask under inert atmosphere were added consecutively: 0.39 g (0.78 mmol) of 3-acetyl-5-dehydroepiandrosterone toluenesulfonyl hydrazone, 0.025 g (0.027 mmol, 3,4 %) of Pd₂(dba)₃, 0.027 g (0.056 mmol, 7.18 %) of XPhos. Thereafter 5 ml of dry dioxane were added to afford a suspension with stirring and 0.11 ml (1.17 mmol, 1.5 eq) of 3-bromopyridine and 0.41 g (5.15 mmol, 6,6 eq) of ^{t}BuOLi were consecutively also added to the suspension. The reaction mixture was heated at reflux for 4 hours, and then it was cooled and filtered, washing the insoluble residue with THF (3 x 5 ml). The filtrates were combined and the solvent was evaporated under reduced pressure to afford a residue wherein abiraterone acetate was detected as one of the main products as confirmed by NMR.

### Example 13.- Synthesis of 3-((tert-butyldimethylsilyl)oxy)abiraterone from 3-((tert-butyldimethysilyl)oxy)-5-dehydroepiandrosterone p-toluenesulfonyl hydrazone

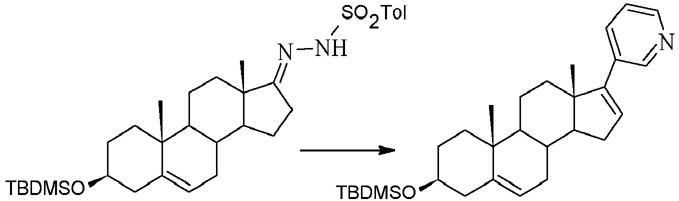

### a) General reaction:

In a round bottom flask under inert atmosphere were added consecutively: 1 g (1.75 mmol) of 3-((tert-butyldimethylsilyl)oxy)-5-dehydroepiandrosterone p-toluenesulfonyl hydrazone, 0.032 g (0.035 mmol, 2 %) of Pd₂(dba)₃, 0.033 g (0.069 mmol, 4 %) of XPhos and 0.35 g (4.37 mmol, 2,5 eq) of t-BuOLi. Then, 30 ml of dry dioxane were added to form a suspension with stirring, over which 0.25 ml (2.62 mmol, 1.5 eq) of 3-bromopyridine were added.

The reaction mixture was heated at reflux for 4 hours, and then it was cooled and filtered, washing the insoluble residue with THF (3 x 5 ml). The filtrates were combined and the solvent was evaporated under reduced pressure to afford a residue containing the desired product (45%), starting material (5%) and a dimeric impurity resulting from two molecules of the starting material (24%).

### b) Using more catalyst

In a round bottom flask under inert atmosphere were added consecutively: 0.57 g (1 mmol) of 3-((tert-butyldimethylsilyl)oxy)-5-dehydroepiandrosterone p-toluenesulfonyl hydrazone, 0.055 g (0.06 mmol, 6 %) of Pd₂(dba)₃, 0.057 g (0.12 mmol, 12 %) of XPhos and 0.2 g (2.5 mmol, 2,5 eq) of t-BuOLi. Then, 15 ml of dry dioxane were added to form a suspension with stirring, over which 0.14 ml (1.50 mmol, 1.5 eq) of 3-bromopyridine were added.

The reaction mixture was heated at 110 °C for 15 hours, and then it was cooled and filtered, washing the insoluble residue with THF (3 x 5 ml). The filtrates were combined and the solvent was evaporated under reduced pressure to afford a residue containing the desired product (43%) and a dimeric impurity resulting from two molecules of the starting material (8.5%).

### c) Using more catalyst and base

In a round bottom flask under inert atmosphere were added consecutively: 0.57 g (1 mmol) of 3-((tert-butyldimethylsilyl)oxy)-5-dehydroepiandrosterone p-toluenesulfonyl hydrazone, 0.055 g (0.06 mmol, 6 %) of Pd₂(dba)₃, 0.057 g (0.12 mmol, 12 %) of XPhos and 0.6 g (7.5 mmol, 7,5 eq) of t-BuOLi. Then, 15 ml of dry dioxane were added to form a suspension with stirring, over which 0.14 ml (1.50 mmol, 1.5 eq) of 3-bromopyridine were added.

The reaction mixture was heated at 110 °C for 15 hours, and then it was cooled and filtered, washing the insoluble residue with THF (3 x 5 ml). The filtrates were combined and the solvent was evaporated under reduced pressure to afford a residue containing the desired product (62%) and a dimeric impurity resulting from two molecules of the starting material (15.4%).

The residue was solved in 10 ml of THF and 17 ml of HCl aq (1M) were added and a suspension was obtained. The solid obtained was filtered at room temperature and washed with water and cold heptane to afford 0.27 g of 3-((tertbutyldimethylsilyl)oxy)abiraterone chlorhydrate (57% yield), free of impurities. Characteristic NMR signals:

¹H NMR (400 MHz, CDCl₃): δ 8.72 (s, 1H), 8.59 (s, 1H), 8.32 (d, J = 7.4 Hz, 1H), 7.82 (s, 1H), 6.34 (s, 1H), 5.35 (d, J = 5.1 Hz, 6H), 3.49 (s, 1H), 1.07 (s, 3H), 1.06 (s, 3H), 0.89 (s, 9H), 0.06 (s, 6H).

### d) Using more catalyst and base as lowering the temperature

In a round bottom flask under inert atmosphere were added consecutively: 0.57 g (1 mmol) of 3-((tert-butyldimethylsilyl)oxy)-5-dehydroepiandrosterone p-toluenesulfonyl hydrazone, 0.055 g (0.06 mmol, 6 %) of Pd₂(dba)₃, 0.057 g (0.12 mmol, 12 %) of XPhos and 0.6 g (7.5 mmol, 7,5 eq) of t-BuOLi. Then, 15 ml of dry dioxane were added to form a suspension with stirring, over which 0.14 ml (1.50 mmol, 1.5 eq) of 3-bromopyridine were added.

The reaction mixture was heated at 90 °C for 15 hours, and then it was cooled and filtered, washing the insoluble residue with THF (3 x 5 ml). The filtrates were combined and the solvent was evaporated under reduced pressure to afford a residue containing the desired product (65%) and a dimeric impurity resulting from two molecules of the starting material (6.4%).

The residue was solved in 10 ml of THF and 17 ml of HCl aq (1M) were added and a suspension was obtained. The solid obtained was filtered at room temperature, washing successively with water and cool hexane, to afford 0.3 g of 3-((tertbutyldimethylsilyl)oxy)abiraterone chlorhydrate (60% yield), with a purity by HPLC of around 95%.

### e) Using a little quantity of water.

In a round bottom flask under inert atmosphere were added consecutively: 0.285 g (0.5 mmol) of 3-((tert-butyldimethylsilyl)oxy)-5-dehydroepiandrosterone p-toluenesulfonyl hydrazone, 0.0275 g (0.03 mmol, 6 %) of Pd₂(dba)₃, 0.0280 g (0.06 mmol, 12 %) of XPhos and 0.6 g (7.5 mmol, 15 eq) of t-BuOLi. Then, 15 ml of dry dioxane were added to form a suspension with stirring, over which 0.072 ml (0.75 mmol, 1.5 eq) of 3-bromopyridine were added. Finally 0.018 ml of water (1 equivalent) were also added.

The reaction mixture was heated at 90 °C for 7 hours, and then it was cooled and filtered, washing the insoluble residue with THF (3 x 5 ml). The filtrates were combined and the solvent was evaporated under reduced pressure to afford a residue containing the desired product (65%) and a dimeric impurity resulting from two molecules of the starting material (6.5%).

### Example 14.- Synthesis of 3-((tert-butyldimethylsilyl)oxy)abiraterone from 3-((tertbutyldimethylsilyl)oxy)-5-dehydroepiandrosterone 2,4,6-trimethylsulfonyl hydrazone

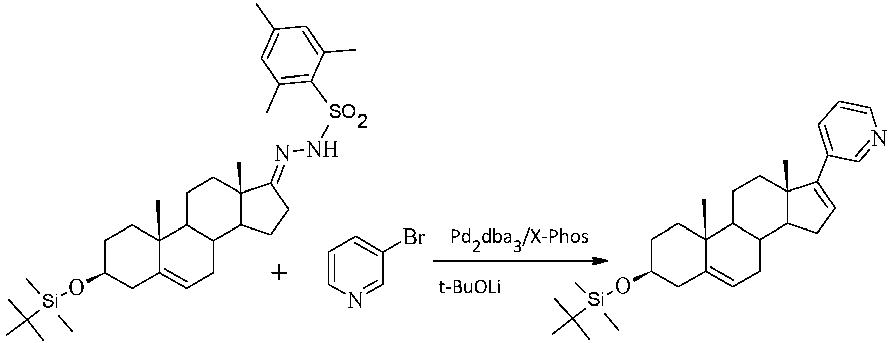

In a round bottom flask under inert atmosphere were added consecutively: 0.299 g (0.5 mmol) of 3-((tert-butyldimethylsilyl)oxy)-5-dehydroepiandrosterone 2,4,6-trimethylsulfonyl hydrazone, 0.0275 g (0.03 mmol, 6 %) of Pd₂(dba)₃, 0.0275 g (0.06 mmol, 12 %) of XPhos and 0.6 g (7.5 mmol, 15 eq) of t-BuOLi. Then, 7.5 ml of dry dioxane were added to form a suspension with stirring, over which 0.072 ml (0.75 mmol, 1.5 eq) of 3-bromopyridine and 0.018 ml of water were added.

The reaction mixture was heated at 90 °C for 6 hours, and then it was cooled and filtered, washing the insoluble residue with THF (3 x 5 ml). The filtrates were combined and the solvent was evaporated under reduced pressure to afford a residue containing the desired product (70%) and a dimeric impurity resulting from two molecules of the starting material of only 3.4%.

The residue was solved in 5 ml of THF and 10 ml of HCl aq (1M) were added and a suspension was obtained. The solid obtained was filtered at room temperature, washing successively with water and cool hexane, to afford 3-((tertbutyldimethylsilyl)oxy)abiraterone chlorhydrate with a purity by NMR of more than 95%.

### Example 15.- Synthesis of 3-((tert-butyldimethylsilyl)oxy)abiraterone from 3-((tertbutyldimethylsilyl)oxy)-5-dehydroepiandrosterone 2,4,6-tri-isopropylsulfonyl hydrazone

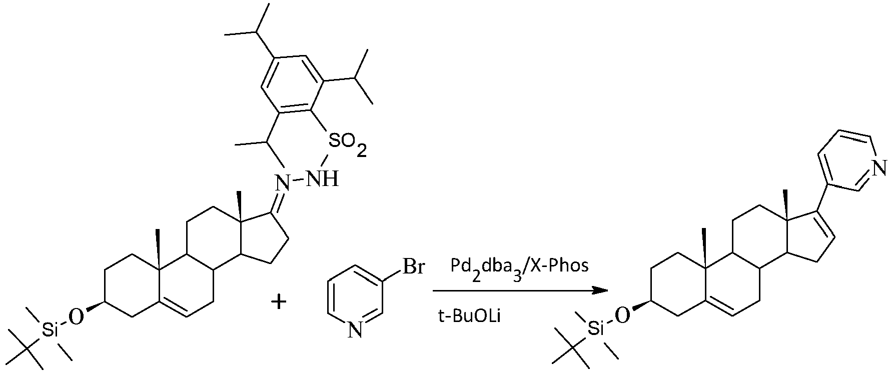

In a round bottom flask under inert atmosphere were added consecutively: 0.170 g (0.25 mmol) of 3-((tert-butyldimethylsilyl)oxy)-5-Ddhydroepiandrosterone 2,4,6-tri-isopropylsulfonyl hydrazone, 0.014 g (0.015 mmol, 6 %) of Pd₂(dba)₃, 0.014 g (0.03 mmol, 12 %) of XPhos and 0.150 g (1.875 mmol, 7.5 eq) of t-BuOLi. Then, 3.75 ml of dry dioxane were added to form a suspension with stirring, over which 0.036 ml (0.375 mmol, 1.5 eq) of 3-bromopyridine were added.

The reaction mixture was heated at 110 °C overnight, and then it was cooled and filtered, washing the insoluble residue with THF (3 x 5 ml). The filtrates were combined and the solvent was evaporated under reduced pressure to afford a residue containing the desired product (60%) and a dimeric impurity resulting from two molecules of the starting material of 15.6 %.

### Example 16.- Synthesis of abiraterone acetate from 3-acetyl-5-dehydroepiandrosterone toluenesulfonyl hydrazone

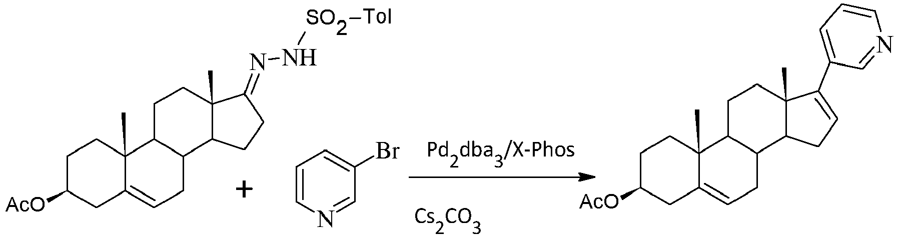

In a round bottom flask under inert atmosphere were added consecutively: 0.25 g (0.5 mmol) of 3-acetyl-5-dehydroepiandrosterone toluenesulfonyl hydrazone, 0.01 g (0.01 mmol, 2 %) of Pd₂(dba)₃, 0.012 g (0.024 mmol, 5 %) of XPhos and 0.36g (2.2 eq) of Cs₂CO₃. Thereafter 7.5 ml of dry dioxane were added to afford a suspension with stirring and 0.072 ml (1.5 eq) of 3-bromopyridine were also added to the suspension. The reaction mixture was heated at 110°C for 5 hours, and then it was cooled and filtered, washing the insoluble residue with THF (3 x 5 ml). The filtrates were combined and the solvent was evaporated under reduced pressure to afford a residue containing abiraterone acetate (10%) and a dimeric impurity resulting from two molecules of the starting material (80%).

### Example 17.- Synthesis of abiraterone acetate from 3-acetyl-5-dehydroepiandrosterone toluenesulfonyl hydrazone

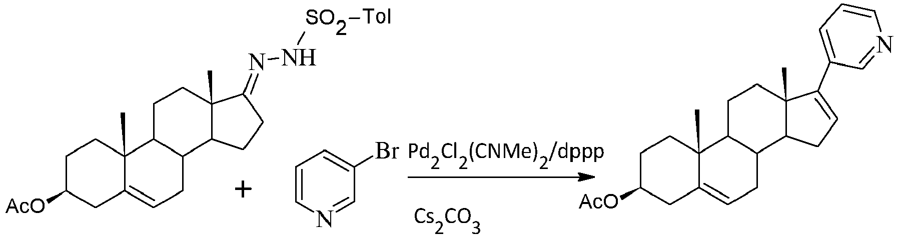

In a round bottom flask under inert atmosphere were added consecutively: 0.124 g (0.25 mmol) of 3-acetyl-5-dehydroepiandrosterone p-toluenesulfonyl hydrazone, 0.003 g (5 %) of PdCl₂(CNMe)₂, 0.01 g (10 %) of dppp and 0.24 g (3 eq.) of Cs₂CO₃. Thereafter 5 ml of dry dioxane were added to afford a suspension with stirring and 0.029 ml (1.5 eq) of 3-bromopyridine were also added to the suspension.

The reaction mixture was heated at 90°C for 52 hours, and then it was cooled and filtered, washing the insoluble residue with THF (3 x 5 ml). The filtrates were combined and the solvent was evaporated under reduced pressure to afford a residue containing abiraterone acetate (15%) and a dimeric impurity resulting from two molecules of the starting material (42%).

### Example 18. Synthesis of 3-((tert-butyldimethylsilyl)oxy)abiraterone from 3-((tertbutyldimethylsilyl)oxy)-5-dehydroepiandrosterone p-toluenesulfonyl hydrazone

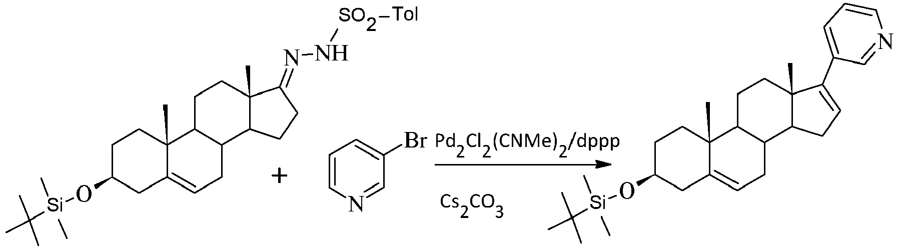

On a round bottom flask under inert atmosphere were added consecutively: 0.570 g (1 mmol) of 3-((tert-butyldimethylsilyl)oxy)-5-dehydroepiandrosterone p-toluenesulfonyl hydrazone, 0.013 g (5 %) of PdCl₂(CNMe)₂, 0.041 g (10 %) of dppp and 0.97 g (3 mmol) of Cs₂CO₃. Thereafter 15 ml of dry dioxane were added to afford a suspension with stirring and 0.144 ml (1.5 eq) of 3-bromopyridine were also added to the suspension.

The reaction mixture was heated at 110°C overnight, and then it was cooled and filtered, washing the insoluble residue with THF (3 x 5 ml). The filtrates were combined and the solvent was evaporated under reduced pressure to afford a residue containing 3-((tert-butyldimethylsilyl)oxy)abiraterone (31%) and a dimeric impurity resulting from two molecules of the starting material (38%).

### Example 19.- Synthesis of abiraterone from 3-((tert-butyldimethylsilyl)oxy)abiraterone

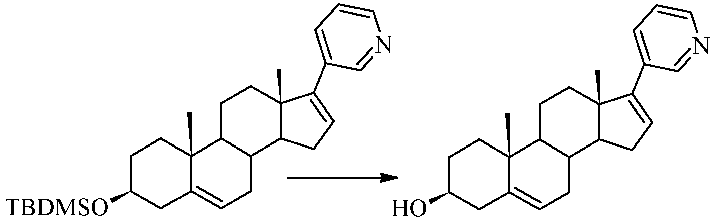

In a round bottom flask were dissolved 0.463 g (1 mmol) of 3-((tertbutyldimethylsilyl)oxy)abiraterone in 10 ml of THF and 7.5 ml of a 1 Molar solution of tetrabutylammonium fluoride in THF was added. The reaction was monitored by TLC until complete deprotection of the hydroxyl group. Once the reaction was complete, the solvent was evaporated partially under reduced pressure and some water was added to the reaction mixture to afford a suspension. The solid formed was isolated by filtration, washed with water (3 x 5 ml) and dried under vacuum to afford 0.31 g (89% yield) of pure abiraterone, as confirmed by NMR.

### Example 20.- Synthesis of abiraterone chlorhydrate from 3-((tertbutyldimethylsilyl)oxy)-5-dehydroepiandrosterone p-toluenesulfonyl hydrazine

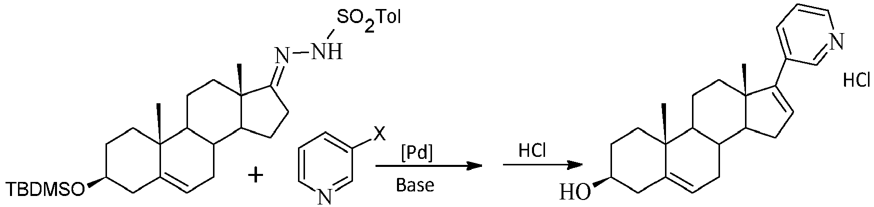

In a round bottom flask under inert atmosphere were added consecutively: 13 g (22.8 mmol) of 3-((tert-butyldimethylsilyl)oxy)-5-dehydroepiandrosterone p-toluenesulfonyl hydrazone, 0.78 g (1.35 mmol) of Pd₂(dba)₃, 1.3 g (2.72 mmol) of XPhos and 17.53 g (219 mmol, 9,5 eq) of t-BuOLi. Then, 195 ml of dioxane were added to form a suspension with stirring, over which 3.25 ml (34.2 mmol) of 3-bromopyridine and 0.39 ml (21.7 mmol) of water were added.

The reaction mixture was heated at reflux for 5 hours, and then it was cooled and filtered. Water (220 ml) and ethyl acetate (220 ml) were added over the filtrate and the two phases formed were decanted, the aqueous phase was extracted again with 100 ml of ethyl acetate and the organic phases were combined and evaporated under reduced pressure to afford a residue containing the desired product.

The residue was solved in 280 ml of isopropyl ether and 18,3 ml of HCl aq (10 M) and 500 ml of water were added. A solid began to appear and a suspension was obtained. The solid obtained was filtered at room temperature, washing successively with water and cool heptane, to afford 6 g of abiraterone chlorhydrate (68% yield), with a purity by HPLC of around 95%.

## Claims

**1.** A process for obtaining a compound of general formula (1) 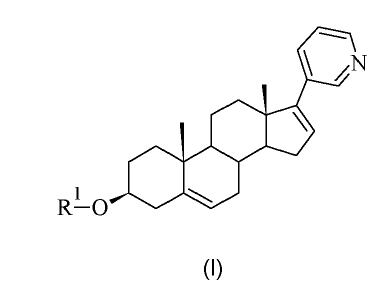 or a salt or solvate thereof,
wherein
R¹ is selected from the group consisting of H, and a hydroxyl protecting group (HPG);
comprising reacting a compound of general formula (II) 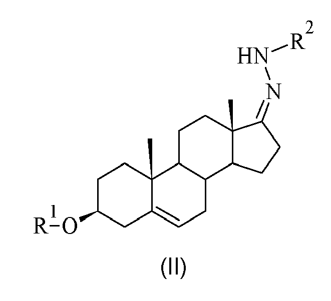 wherein
R¹ has the previously mentioned meanings, R² is SO₂R⁷ and R⁷ is selected from the group consisting of optionally substituted C₁-C₈ alkyl and optionally substituted C₆-C₁₄ aryl;
with a compound of formula (III) 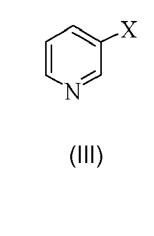 wherein
X is halogen or OSO₂CF₃;
in the presence of a palladium catalyst and a base.

**2.** The process according to any one of the preceding claims, wherein the palladium catalyst is selected from Pd₂(dba)₃, Pd(PPh₃)₄, PdCl₂(CNMe)₂, Pd(OH)₂ and Pd(OAc)₂.

**3.** The process according to any one of the preceding claims, further comprising the addition of a ligand to the reaction media, said ligand being preferably selected from X-phos (2-dicyclohexylphosphino-2',4',6'-triisopropylbiphenyl), dppp (1,4-bis(diphenylphosphino)butane), S-phos (2-dicyclohexylphosphino-2',6'-dimethoxybiphenyl), dppm (1,1-bis(diphenylphosphino)-methane), dippe (1,2-bis(diisopropylphosphino)ethane, dmpe (1,2-Bis(dimethylphosphino)ethane and dppe (1,2-bis(diphenylphophino)ethane.

**4.** The process according to any one of the preceding claims, wherein the base is selected from alkoxides and carbonates of alkaline and alkaline earth metals, preferably from t-BuOLi, MeOLi, MeONa and CsCO₃.

**5.** The process according to any one of the preceding claims, wherein R² is selected from the group consisting of SO₂Ph, SO₂Tol, SO₂(2,4,6-trimethylphenyl) and SO₂(2,4,6-triisopropylphenyl).

**6.** The process according to any one of the preceding claims, wherein the compound
of general formula (II) is prepared by reacting a ketone of general formula (VI) 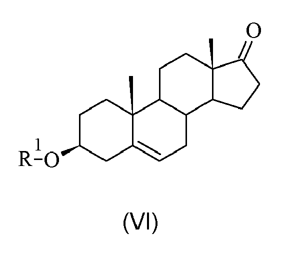 wherein
R¹ is selected from the group consisting of H and a hydroxyl protecting group (HPG);
and a hydrazine of general formula (VII): 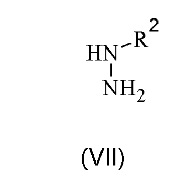 wherein
R² is SO₂R⁷ and R⁷ is selected from the group consisting of optionally substituted C₁-C₈ alkyl and optionally substituted C₆-C₁₄ aryl.

**7.** A process for obtaining a compound of general formula (I) 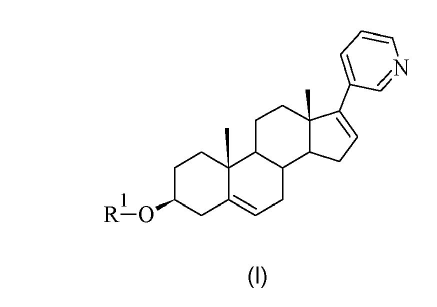 or a salt or solvate thereof,
wherein
R¹ is selected from the group consisting of H and a hydroxyl protecting group (HPG);
comprising reacting a compound of general formula (VI) 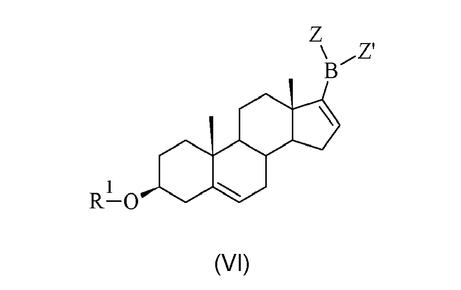 wherein
R¹ is selected from the group consisting of H and a hydroxyl protecting group (H PG); and
Z and Z' are independently selected from the group consisting of hydroxyl, optionally substituted C₁-C₈ alkoxy and optionally substituted C₁-C₈ alkyl, or Z and Z' together form an optionally substituted C₂-C₃ alkylenedioxy group;
with a compound of formula (III) 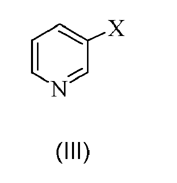 wherein
X is halogen or OSO₂CF₃.
in the presence of a palladium catalyst and a base.

**8.** The process according to claim 7 wherein the palladium catalyst is selected from Pd(PPh₃)₄ and Pd(PPh₃)₂Cl₂.

**9.** The process according to any one of claims 7 to 8, wherein the base is selected from sodium, cesium and potassium carbonate.

**10.** The process according to any one of the preceding claims, wherein R¹ is selected from the group consisting of H, COMe, SitBuMe₂ (TBDMS) and SiPhMe₂ (DMPS).

**11.** The process according to any one of the preceding claims, wherein the compound of general formula (III) is 3-bromopyridine.

**12.** The process according to any one claims 7-10, wherein the compound of general
formula (IV) is prepared by reacting a compound of general formula (IIa) 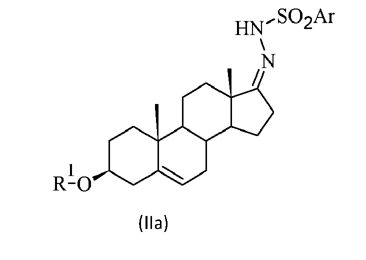 wherein
R¹ is selected from the group consisting of H and a hydroxyl protecting group (HPG); and
Ar is optionally substituted C₆-C₁₄ aryl; or
a compound of general formula (IX) 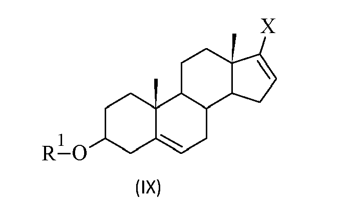 wherein
R¹ is selected from the group consisting of H and a hydroxyl protecting group (HPG); and
X is bromo or iodo;
with a lithium base and a compound of formula (VIII) 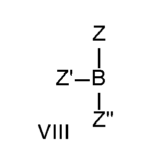 wherein
Z, Z' and Z" are independently selected from the group consisting of hydroxyl, optionally substituted C₁-C₈ alkoxy and optionally substituted C₁-C₈ alkyl, or
Z and Z' together form a optionally substituted C₂-C₃ alkylenedioxy group and Z" is selected from the group consisting of hydroxyl, optionally substituted C₁-C₈ alkoxy and optionally substituted C₁-C₈ alkyl.

**12.** The process according to any one of the preceding claim, further comprising the transformation and/or purification of the compound of general formula (I) obtained in other compound of general formula (I), said transformation and/or purification comprising one or more of the following steps:
i) purification of a compound of formula (I) by means of crystallization and/or salt formation;
ii) transformation of the compound of formula (I) to abiraterone (R¹=H), by means of a deprotection reaction which, depending on the nature of group R₁, comprises:
a) hydrolysis in acid or basic media,
b) use of fluoride reagents, or
c) oxidation or reduction;
iii) esterification of abiraterone (R¹=H) to afford abiraterone acetate.

**13.** A compound of general formula (II) 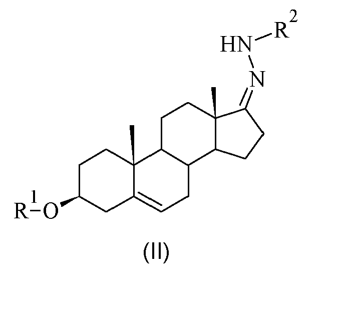 or a salt or solvate thereof, wherein
R¹ is selected from the group consisting of H and a hydroxyl protecting group (H PG)
R² is SO₂R⁷
R⁷ is selected from the group consisting of optionally substituted C₁-C₈ alkyl and optionally substituted C₆-C₁₄ aryl.

**14.** A compound of general formula (IV) 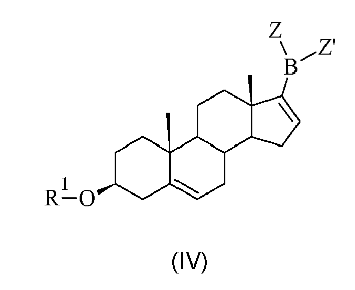 or a salt or solvate thereof, wherein
R¹ is selected from the group consisting of H and a hydroxyl protecting group (H PG); and
Z and Z' are independently selected from the group consisting of hydroxyl, optionally substituted C₁-C₈ alkoxy and optionally substituted C₁-C₈ alkyl, or Z and Z' together form a optionally substituted C₂-C₃ alkylenedioxy group.

**15.** A compound of general formula (I) 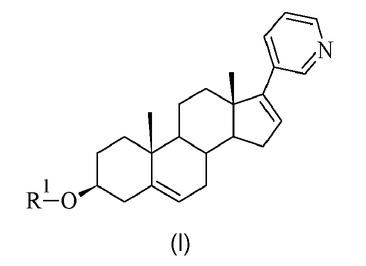 or a salt or solvate thereof, wherein
R¹ is SiR³R⁴R⁵. and
R³, R⁴ and R⁵ are independently selected from the group consisting of optionally substituted C₁-C₈ alkyl, optionally substituted C₃-C₆ cycloalkyl, optionally substituted C₆-C₁₄ aryl, optionally substituted C₁-C₈ alkoxy, and halogen.

**16.** A process for the preparation of a salt of a compound of formula (I) as defined in claim 15 by recovering the salt from a solution of the free base in any suitable solvent by treating the solution with an appropriate acid, wherein preferably the compound of formula (I) is 3-TBDMS-abiraterone and/or
the acid is hydrochloric acid so that the salt is the chlorhydrate salt.
